# EUROPEAN PATENT APPLICATION

(11) **EP 2 286 717 A1**
(43) Date of publication of application: **23.02.2011**
(21) Application number: 10173084.4
(22) Date of filing: 17.08.2010
(51) Int. Cl.: A61B 1/01, A61B 1/012, A61B 1/018, A61B 17/34

(54) **Insertion path securing apparatus and mantle tube**

(30) Priority: 18.08.2009 JP 2009189444
(71) Applicant: Fujifilm Corporation, Minato-ku Tokyo (JP)
(72) Inventor: MATSUSHITA, Motohiko, Kanagawa (JP)
(74) Representative: Klunker . Schmitt-Nilson . Hirsch

(57) **Abstract**

It is an insertion path securing apparatus for guiding an insertion instrument to be inserted into a body-cavity, by an insertion portion having a longitudinal axis. The insertion path securing apparatus includes a first bending member configured to be bendable by operating a first bending operation portion, a second bending member configured to be bendable at a position differing from that of the first bending member by operating a second bending operation member, and a hardness changing member. A hardness variable portion of the hardness changing member can change hardness between a flexible state in which flexibility, with which the insertion portion is bendable by the first or second bending member, is given to the insertion portion by operating the hardness changing operation portion, and a rigid state in which a curved shape given to the insertion portion by the first and second bending members is maintained.

## Description

### BACKGROUND OF THE INVENTION

### 1. Technical Field

The present invention relates to an insertion path securing apparatus for guiding an insertion instrument that is inserted into a body-cavity, and to a mantle tube.

### 2. Description of Related Art

Securing an insertion path through which various medical instruments such as an endoscope are inserted into a body-cavity has been performed by inserting a mantle tube into the body-cavity when the observation and the treatment of the inside of the body-cavity. In addition, mantle tubes also are utilized when the treatment of a specific intracavital region is performed with low invasiveness by incising a body-cavity tube wall or skin to form a small opening. There is a mantle tube of such a type that has a function of transmitting torque for bending thereof and that can be fixed in a shape bent with a vacuum suction force supplied externally (see, US 2008/0039691 A). Either a state in which the shape of the mantle tube is flexibly variable, or another state in which the mantle tube is fixed by maintaining the shape thereof, can be selected as the state thereof.

On the other hand, the following mantle tubes are provided (see JP-A-2005-46275). That is, a double tube structure is obtained by overlapping two mantle tubes, each of which can select a shape variable state and a fixed state, one on the other. One of the mantle tubes, which is brought into a shape variable state, is advanced relatively with respect to the other mantle tube put into a fixed state. Thus, the mantle tubes are alternately advanced into an insertion path to follow the shape of the inside of a body-cavity.

According to the above mantle tubes, the insertion of various medical instruments into insertion paths is facilitated, even though the insertion paths are complex ones provided in the body-cavity. When a plurality of curved-portions is formed in the insertion path, it is difficult to insert the mantle tube into the body-cavity by surely fixing a shape at the position of each curved-portion. That is, the curved shape of a leading-edge portion of the inserted mantle tube can be followed by the medical instrument. However, in a case where the curved-shape of an already bent part closer to the base-end-side than the leading edge-portion thereof is once followed, the curved-shape cannot be maintained without change. The curved shape is changed while an insertion operation is continued. When the inner and outer mantle tubes of a mantle pipe having the double tube structure described in JP-A-2005-46275 are alternately fixed, the curved shape gradually is uncurved. Thus, the initial curved shape cannot be maintained.

### SUMMARY

An object of the invention is to provide an insertion path securing apparatus capable of securing an insertion path having a desired shape while surely maintaining the shape of a mantle tube once bent, and to provide the mantle tube.

[1] It is an insertion path securing apparatus for guiding an insertion instrument to be inserted into a body-cavity by using an insertion portion having a longitudinal axis. The insertion path securing apparatus includes a first bending member, a second bending member and a hardness changing member. The first bending member is arranged advanceably and withdrawably along the longitudinal axis of the insertion portion. The first bending member has a first bending action portion provided at a distal side and a first bending operation portion provided at a proximal side, and can bend the first bending action portion by operating the first bending operation portion. The second bending member is arranged advanceably and withdrawably along the longitudinal axis of the insertion portion. The second bending member has a second bending action portion provided at a distal side and a second bending operation portion provided at a proximal side, and can bend the second bending action portion at a position on the longitudinal axis, which differs from a position corresponding to the first bending member, by operating the second bending operation portion. The hardness changing member is arranged along the longitudinal axis of the insertion portion, has a hardness variable portion provided at a distal side and a hardness changing operation portion provided at a proximal side. The hardness variable portion is configured to be able to change hardness between a flexible state and a rigid state by operating the hardness changing operation portion. In the flexible state, flexibility is imparted to the insertion portion by operating the hardness changing operation portion to be able to bend the insertion portion by the first bending member or the second bending member. In the rigid state, a curved shape imparted to the insertion portion by the first bending member or the second bending member is maintained.

[2] It is a mantle tube having an operating portion provided at a base-end side of an insertion portion having a longitudinal axis. The mantle tube includes a first lumen, a second lumen and a third lumen. A first bending member can be provided in the first lumen to have a first bending action portion provided at a distal side and a first bending operation portion provided at a proximal side, and to be able to bend the first bending action portion by operating the first bending operation portion. A second bending member can be provided in the second lumen to have a second bending action portion provided at a distal side and a second bending operation portion provided at a proximal side, and to be able to bend the second bending action portion at a position differing from that of the first bending member on the longitudinal axis by operating the second bending operation portion. A hardness changing member can be provided in the third lumen to have a hardness variable portion provided at a distal side and a hardness changing operation portion provided at a proximal side. The hardness variable portion is configured to be able to change hardness between a flexible state and a rigid state by operating the hardness changing operation portion. In the flexible state, flexibility is imparted to the insertion portion by operating the hardness changing operation portion to be able to bend the insertion portion by the first bending member or the second bending member. In the rigid state, a curved shape imparted to the insertion portion by the first bending member or the second bending member is maintained.

[3] It is a mantle tube having an operating portion provided at a base-end side of an insertion portion having a longitudinal axis. The mantle tube includes a first lumen and a second lumen. A first bending member can be removably provided in the first lumen to have a first bending action portion provided at a distal side and a first bending operation portion provided at a proximal side, and to be able to bend the first bending action portion by operating the first bending operation portion. A second bending member can be removably provided in the second lumen to have a second bending action portion provided at a distal side and a second bending operation portion provided at a proximal side, and to be able to bend the second bending action portion at a position differing from that of the first bending member on the longitudinal axis by operating the second bending operation portion. The insertion portion is a mantle tube whose hardness can be changed between a flexible state and a rigid state by operating the hardness changing operation portion. In the flexible state, the insertion portion has flexibility so that the insertion portion can be bent by the first bending member or the second bending member. In the rigid state, a curved shape imparted to the insertion portion by the first bending member or the second bending member is maintained.

The insertion path securing apparatus and the mantle tube according to the invention can surely secure an insertion path having a desired shape while maintaining the shape of the mantle tube, which is once bent.

### BRIEF DESCRIPTION OF THE DRAWINGS

FIG. 1 is a diagram for illustrating an embodiment of the invention, which is a view showing the configuration of the entire insertion path securing apparatus.
FIG. 2 is a partially enlarged diagram illustrating a mantle tube illustrated in FIG. 1.
FIG. 3 is a cross-sectional diagram taken on line A-A illustrated in FIG. 2.
FIG. 4 is a diagram illustrating the configuration of the mantle tube, and showing a state in which a valve element is provided in each of a guide hole and communication holes in a base-end portion of the mantle tube.
FIG. 5 is a diagram schematically illustrating the external-appearance configuration of an endoscope.
FIG. 6 is a diagram illustrating an external-appearance configuration of a bending jig.
FIGS. 7A to 7F are explanatory diagrams respectively illustrating steps of a transvaginal procedure that is an example of a procedure using an insertion path securing apparatus. FIG. 7A illustrates a state when an incision is performed. FIG. 7B illustrates a state when the insertion path securing apparatus is inserted. FIG. 7C illustrates a state when tissue-expansion is performed. FIG. 7D illustrates a state when a lesion location is checked. FIG. 7E illustrates a state when a diseased part is excised. FIG. 7F illustrates a state when the diseased part that is excised is retrieved.
FIG. 8 is an explanatory diagram illustrating a curved shape of the insertion path securing apparatus.
FIG. 9 is an explanatory diagram illustrating a first insertion path securing procedure.
FIG. 10 is another explanatory diagram illustrating the first insertion path securing procedure.
FIG. 11 is an explanatory diagram illustrating a second insertion path securing procedure.
FIG. 12 is another explanatory diagram illustrating the second insertion path securing procedure.
FIG. 13 is an explanatory diagram illustrating a third insertion path securing procedure.
FIG. 14 is an explanatory diagram illustrating a fourth insertion path securing procedure.
FIG. 15 is an explanatory diagram illustrating a fifth insertion path securing procedure.
FIG. 16 is a cross-sectional diagram taken at a leading end portion of a mantle tube, which is at a side to be inserted into a body-cavity.
FIG. 17 is a partially cross-sectional perspective diagram taken at the leading end portion of the mantle tube, which is at the side to be inserted into the body-cavity.
FIG. 18 is a cross-sectional diagram illustrating the mantle tube.
FIG. 19 is a perspective diagram illustrating a leading end portion of a bending sheath member of a bending jig.
FIG. 20 is a cross-sectional diagram illustrating a mantle tube into which the bending sheath member illustrated in FIG. 19 is inserted.
FIG. 21 is a cross-sectional diagram illustrating the mantle tube into which the bending sheath member illustrated in FIG. 19 is inserted.
FIG. 22 is a perspective diagram illustrating a leading end portion of the bending sheath member of the bending jig.
FIG. 23 is a cross-sectional diagram illustrating a mantle tube into which the bending sheath member illustrated in FIG. 22 is inserted.

### DETAILED DESCRIPTION OF EMBODIMENTS OF THE INVENTION

Hereinafter, an embodiment of the invention is described in detail with reference to the drawings.

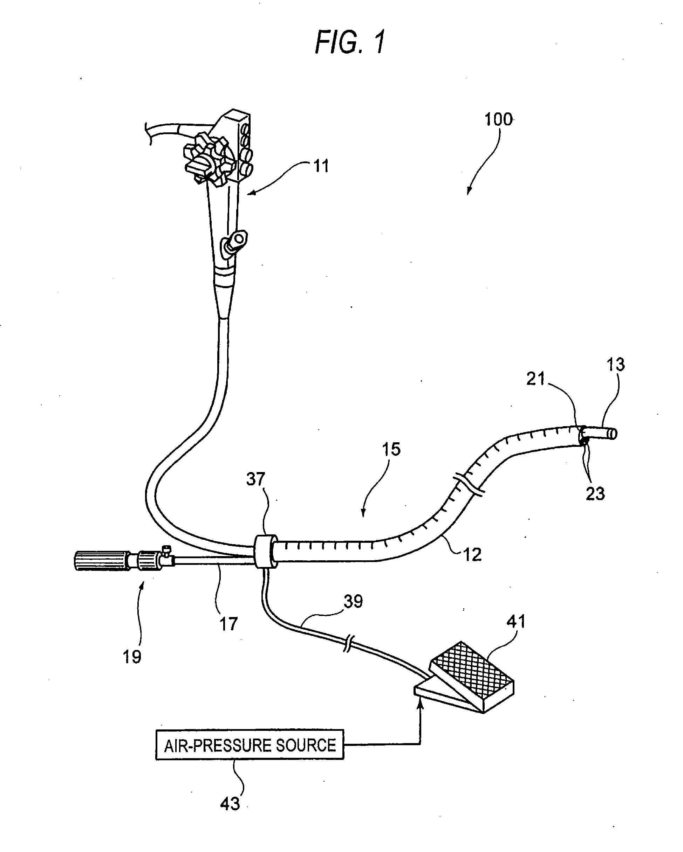

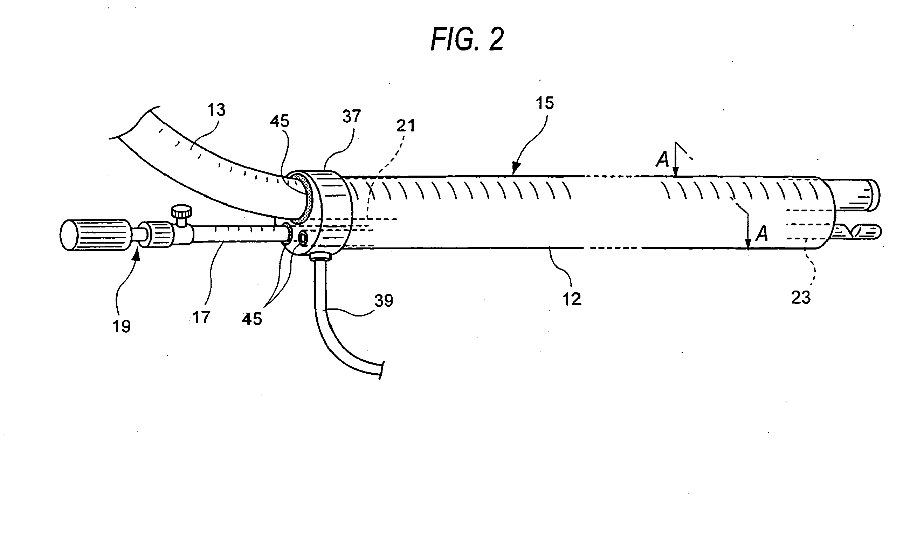

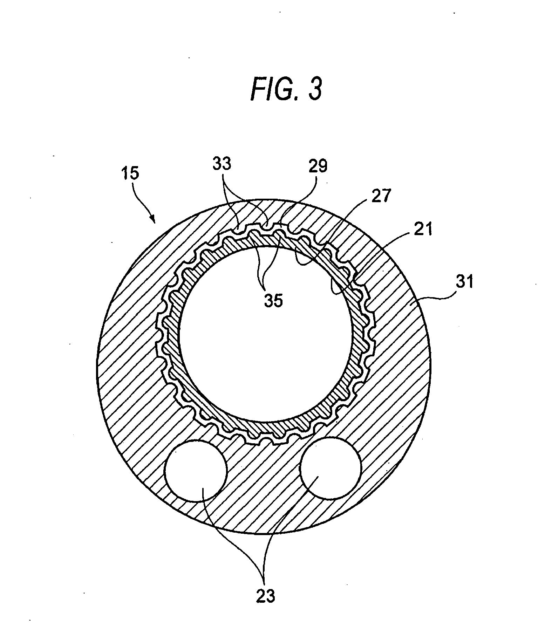

FIG. 1 is a diagram for illustrating an embodiment of the invention, which is a view showing the configuration of the entire insertion path securing apparatus. FIG. 2 is a partially enlarged diagram illustrating a mantle tube illustrated in FIG. 1. FIG. 3 is a cross-sectional diagram taken on line A-A shown in FIG. 2.

As illustrated in FIG. 1, an insertion path securing apparatus 100 is used for guiding insertion instruments (various medical instruments such as an endoscope and a treatment tool, particularly, an endoscope insertion portion 13 of an endoscope 11) to be inserted into a body-cavity. The insertion path securing apparatus 100 includes an elongated tube-like mantle tube 15, and a bending jig (bending member) 19 having a bending sheath portion 17 that has a bendable bending portion and is arranged along a longitudinal direction of the mantle tube 15.

As illustrated in FIG. 2, the mantle tube 15 includes an insertion portion 12 having a longitudinal axis, and an operating portion 37 provided at the base-end side of the insertion portion 12. The insertion portion 12 has openings provided at both end portions thereof and a guide hole 21 extending along a longitudinal direction thereof to communicate therewith. An insertion tool is insertably and removably passed in the guide hole 21. A communication hole 23 into which a the bending sheath portion 17 is inserted is formed along a longitudinal direction in the insertion portion 12. The bending sheath portion 17 is insertably and removably passed in the communication hole 23. That is, the insertion portion 12 has a multi-lumen structure in which a plurality of lumens are formed. Preferably, to facilitate observation with an endoscope, at least a leading end portion of the insertion portion 12, which is at a side to be inserted into a body-cavity, is formed of a flexible translucent material such as an acrylic resin, a polyethylene resin, and a polyvinyl-chloride resin.

As illustrated in FIG. 3, the insertion portion 12 of the mantle tube 15 includes an inner sleeve 27 whose inner circumferential surface is formed as the guide hole 21, and an outer sleeve 31 accommodating the inner sleeve 27 via a hollow space 29 provided between the outer sleeve 31 and an outer circumferential surface of the inner sleeve 27. A plurality of support protrusions 33 are formed on the inner circumferential surface of the outer sleeve 31 to protrude therefrom, and a plurality of support protrusions 35 are formed on the outer circumferential surface of the inner sleeve 27 to protrude therefrom so that each of the support protrusions 33 faces an associated one of the support protrusions 35 in an associated radial direction of the guide hole 21. Both end portions of the inner sleeve 27, which are arranged in the longitudinal direction of the mantle tube 15, are airtightly fixed to end portions of the outer sleeve 31, respectively.

A pressure supply duct 39 communicating with the hollow space 29 is connected to the operating portion 37 of the mantle tube 15. The pressure supply duct 39 is such that a negative pressure from an air pressure source 43 can be supplied to the hollow space 29 by a foot switch 41 illustrated in FIG. 1.

The mantle tube 15 of the above configuration operates as follows. That is, when the pressure of the hollow space 29 between the outer circumferential surface of the inner sleeve 27 and the inner circumferential surface of the outer sleeve 31 is substantially equal to atmospheric pressure, the outer circumferential surface of the inner sleeve 27 and the inner circumferential surface of the outer sleeve 31 are separated from each other. When the pressure of the hollow space 29 is a negative pressure, the inner sleeve 27 which is thin, as compared with the outer sleeve 31, is deformed in a diameter increasing direction and attached close to the inner circumferential surface of the outer sleeve 31. When the inner sleeve 27 is closely attached thereto, each of the support projections 35 formed on the inner sleeve 27 is fit into between an associated pair of the support protrusions 33 and 33 formed on the outer sleeve 31. The bending rigidity (referred to also as hardness) of the insertion portion 12 of the mantle tube 15 increases, so that the shape of the entire insertion portion 12 is fixed. On the other hand, when the pressure of the hollow space 29 is substantially equal to atmospheric pressure, the insertion portion 12 of the mantle tube 15 exhibits bending rigidity sufficient to the extent that the insertion portion 12 is flexible and bendable.

That is, the mantle tube 15 functions as a hardness changing member capable of changing the hardness of the insertion portion 12. The bending rigidness of the insertion portion 12 can be changed between a flexible state, in which the insertion portion 12 exhibits a bending rigidity sufficient to the extent that the insertion portion 12 is flexible, and a rigid state, in which the shape of the insertion portion 12 is fixed, by operating the foot switch 41 serving as a hardness changing operation means attached to the pressure supply duct 39 that is connected to the operating portion 37 of the mantle tube 15. The foot switch 41 functions as a state maintaining means that maintains the once-changed bending rigidity of the mantle tube 15, until the foot switch 41 is operated again.

The configuration of changing the bending rigidity of the mantle tube 15 is described in, e.g., JP-T-5-503434. In addition, known shape fixing mechanisms described in JP-A-57-209032 and JP-T-2006-505302 and the like can be applied to the apparatus.

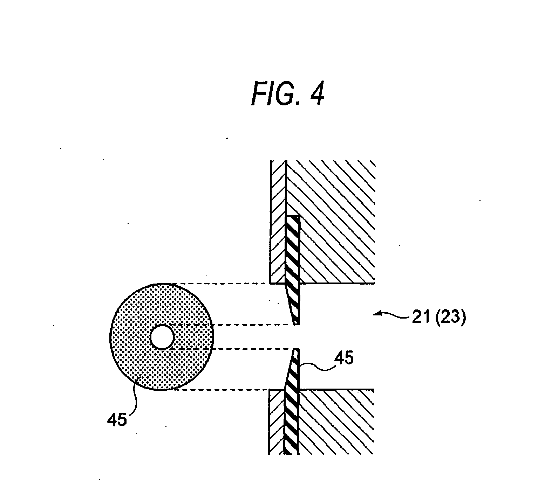

As illustrated in FIG. 4, a thin rubber valve element 45 for preventing, when an insertion instrument such as the endoscope 11 and the bending sheath portion 17 are inserted into a body-cavity, air from flowing into and out of the duct is provided in each of the guide hole 21 and the communication holes 23 formed in the base-end portion of the mantle tube 15. The intra-abdominal air-pressure of the body-cavity, into which the insertion tool and the like are inserted, is prevented from being changed.

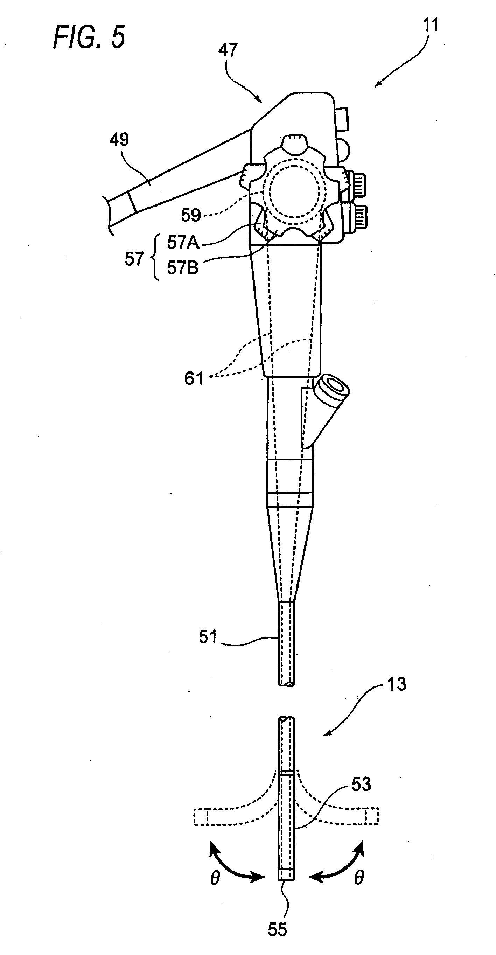

FIG. 5 is a diagram schematically illustrating the external-appearance configuration of the endoscope 11.

The endoscope 11 includes a main-body operating portion 47, and an endoscope insertion portion 13 that is provided continuously to the main-body operating portion 47 and inserted into the body-cavity. A universal chord 49 connected to the main-body operating portion 47 is connected to a light source apparatus (not shown) and a signal processing apparatus (not shown) to perform input/output of illuminating light and an imaging signal.

The endoscope insertion portion 13, whose surfaces are coated with a resin material, includes a flexible portion 51, a bending portion 53, and a leading end portion (endoscope leading-end portion) 55, which are arranged in this order from the side of the main-body operating portion 47. The bending portion 53 serves as a bending action portion to remotely be operated by turning a bending operation portion 57 (angle knobs 57A and 57B) of the main-body operating portion 47. More specifically, a pulley 59 is coaxially provided on a rotating shaft of the angle knobs 57A and 57B. An operating wire 61 wound around the pulley 59 between the pulley 59 and the leading end portion 55 of the endoscope is disposed along the inner wall of the endoscope insertion portion 13. The operating wire 61 is fixed to the leading portion 55 of the endoscope by fixing both ends thereof thereto. Consequently, the operating wire 61 is pulled by performing a turning operation on each of the angle knobs 57A and 57B. Thus, the bending portion 53 is bent, so that the leading end portion 55 of the endoscope can be directed to a desired direction.

In an illustrated example, only a single system for bending the bending portion 53 in a direction of an angle θ corresponding to the angle knob 57A is shown. However, another system for bending the bending portion 53 in a direction perpendicular to the above direction of the angle θ (direction perpendicular to a page plane of FIG. 5) is incorporated in the main-body operating portion 47 and the endoscope insertion portion 13. That is, the leading end portion 55 of the endoscope can freely been bent in a lateral direction and an up-down direction perpendicular to the lateral direction by operating the angle knobs 57A and 57B. As described below, the bending portion 53 functions as a bending member.

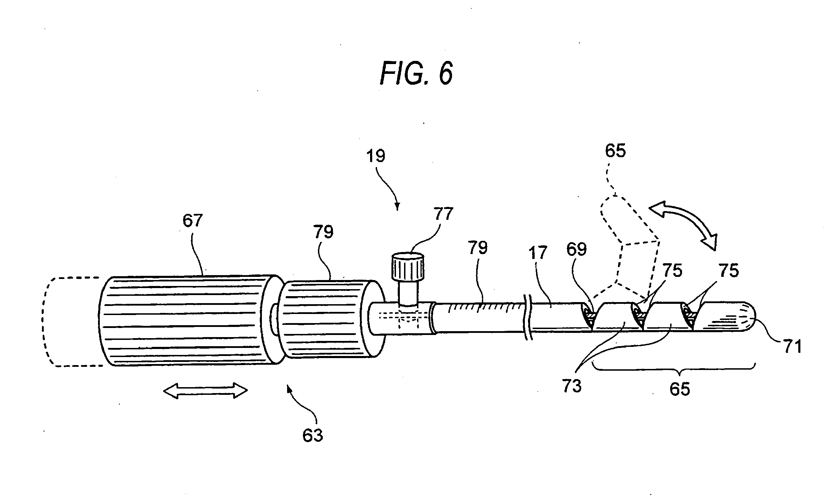

FIG. 6 illustrates an external-appearance configuration of the bending jig 19.

The bending jig 19 includes a bending operation portion 63, a hollow elongated bending sheath portion 17 extended from the bending operation portion 63 to have the bendable bending portion 65, and a traction wire 69 inserted into the bending sheath portion 17 so that an end thereof is fixed to a leading end portion of the bending sheath portion 17 and that the other end thereof is fixed to an operating handle 67 of the bending operation portion 63. The bending portion 65 serving as the bending action portion includes a leading-end piece 71 and a plurality of bending pieces 73 provided in a connected-row-arrangement. Opposed end surfaces of the pieces facing each other have cutout portions 75 each of which has an obliquely cutaway part. The pieces 71 and 73 are connected at positions corresponding to the same side with respect to the central axis of the bending sheath portion 17. A fixing screw 77 to be pressed against the traction wire 69 to fix the wire 69 is provided at the base-end portion of the bending sheath portion 17, which is provided at the side of the bending operation portion 63.

According to the bending jig 19 of the above configuration, the operating handle 67 of the bending operation portion 63 is operated to be separated from a fixing portion 79. Thus, the traction wire 69 is pulled out of the bending sheath portion 17. An operation of bending the bending portion 65 is performed until the end surfaces of the cutout portions 75 of the leading end piece 71 and the bending piece 73 abut against each other. The traction wire 69 is fixed by tightening the fixing screw 77 serving as a state maintaining means. Thus, the curved state of the curved shape of the bending sheath portion 17 can be maintained. When the fixing screw 77 is loosened, the curved state thereof can be terminated.

Scale marks 79 are provided along a longitudinal direction on the outer circumferential surface of the bending sheath portion 17. Thus, the length of an inserted part of the bending sheath portion 17, i.e., a position in a longitudinal direction thereof, to which the bending sheath portion 17 is inserted into the mantle tube 15, can be read. The bending sheath portion 17 is inserted into the communication hole 23 of the mantle tube 15 illustrated in FIG. 3 and bends the mantle tube 15. However, the guide hole 21 of the mantle tube 15 is eccentrically shifted from the central axis of the mantle tube 15. The thickness of a part of the mantle tube 15, which is at the side eccentrically shifted to the guide hole 21, is thin. Accordingly, the bending of the mantle tube 15 is facilitated.

Next, an example of a manipulation of inserting an endoscope from a natural hole such as a mouth, an anus, and a vagina, provided in a human body using the insertion path securing apparatus 100 of the above configuration, adding a small incision to a lumen, and performing diagnosis and treatment when the endoscope reaches a body-cavity. This manipulation is a low-invasiveness surgery capable of reducing or eliminating incisions in an abdominal wall, and called a natural orifice translumental endoscopic surgery (NOTES).

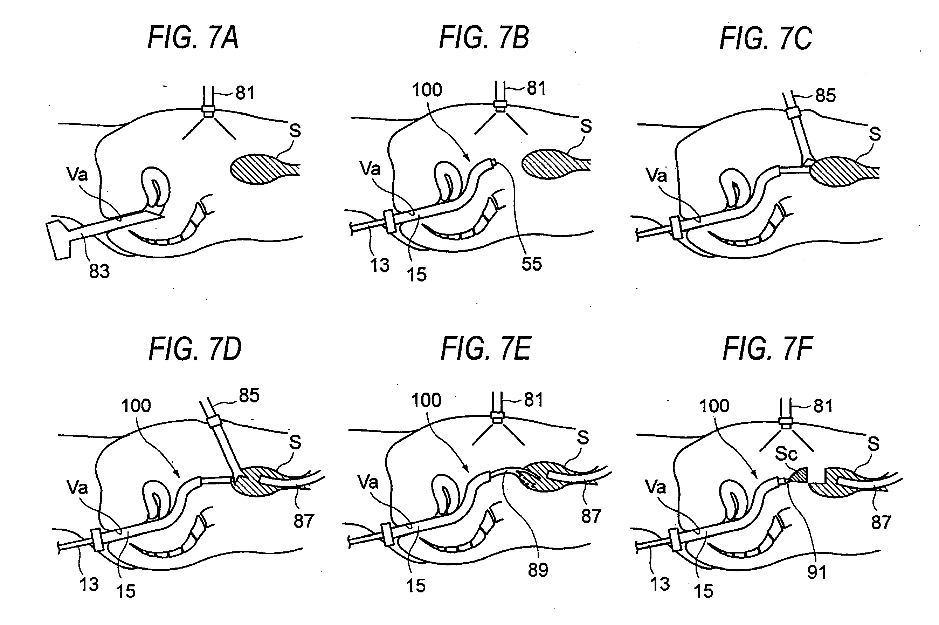

FIGS. 7A to 7F illustrate, in stages, an example of a procedure for performing a transvaginal manipulation (transvaginal extraction of a gastric tumor) using the present insertion path securing apparatus 100.

First, a laparoscope 81 is inserted via a patient's peritoneal wall. A trocar 83 for incising a body-surface tissue is inserted from the patient's vagina Va. As illustrated in FIG. 7A, the trocar 83 is caused to reach a posterior vaginal fornix. A part of the trocar 83 is inserted into a body-cavity by incising a tissue while checking with the laparoscope 81.

As illustrated in FIG. 7B, the trocar 83 is withdrawn from the patient's vagina Va. Then, as illustrated in FIG. 2, the insertion path securing apparatus 100 brought into a state, in which an endoscope (endoscope insertion portion 13) is inserted into the guide hole 21 of the mantle tube 15, is inserted from the patient's vagina Va.

After it is confirmed with the laparoscope 81 that the mantle tube 15 and the leading end portion 55 of the endoscope are located at appropriate positions, the laparoscope 81 is withdrawn.

As illustrated in FIG. 7C, an exclusion device 85 is inserted from a port provided in a peritoneal wall, into which the laparoscope 81 is inserted. A path for the insertion path securing apparatus 100 is secured by moving a tissue located on the path therefor.

If necessary, an endoscope 87 is advanced into a stomach S through the mouth, as illustrated in FIG. 7D. Thus, a lesion location in the stomach S is checked.

A high-frequency cutting instrument is inserted via a forceps port of the endoscope to incise a surrounding tissue that supports the stomach S. Thus, the stomach S is put into a movable state. Then, the inserted endoscope 87 is withdrawn. As illustrated in FIG. 7E, a stapler 89 is inserted into the empty guide hole 21 of the mantle tube 15. A diseased part is excised under observation by the laparoscope 81.

The stapler 89 is withdrawn from the mantle tube 15. As illustrated in FIG. 7F, the endoscope 87 is inserted into the empty guide hole 21 of the mantle tube 15 again. Under observation by the laparoscope 81, the excised diseased-part Sc is grabbed by a retrieving forceps 91 inserted into the forceps port of the endoscope 87. Then, the diseased part is retrieved to the outside of the body-cavity.

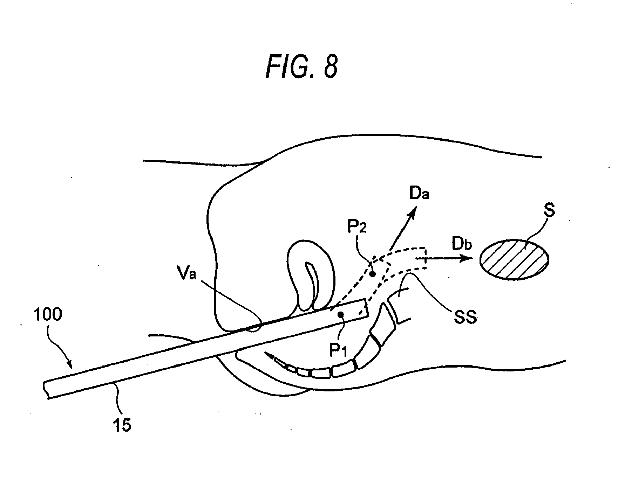

When the above manipulation described as an example is performed, if the mantle tube 15 of the insertion path securing apparatus 100 illustrated in FIG. 8 is advanced along an insertion direction in the vagina Va, a leading end of the mantle tube 15 may touch or press the patient's sacrum SS, particularly, a protruded part thereof, which is called a "promontory". Thus, preferably, as indicated by dashed lines in FIG. 8, the mantle tube 15 is bent to avoid the sacrum SS. At that time, the stomach S serving as a target diseased part is not located in a direction Da to which the leading end of the bent mantle tube 15 is directed, but a direction Db differing from the direction Da. Therefore, as indicated by alternate long and short dash lines in FIG. 8, the mantle tube 15 needs bending one more time.

Thus, according to the present insertion path securing apparatus 100, when the mantle tube 15 is bent, a bending function member is disposed at each bending point to surely maintain the curved shape of the mantle tube 15 on which a plurality of bending points are formed. Consequently, the invention imparts, to the insertion path securing apparatus 100, the function of surely maintaining the curved shape at each bending point halfway through the formation of a curved shape until the shape of the mantle tube 15 is fixed to a final curved shape.

Hereinafter, an example of the procedure for securing an insertion path by the insertion path securing apparatus 100 is described sequentially. A manner of securing an insertion path in the case of a transvaginal extraction of a gastric tumor is described below.

### <First Insertion Path Securing Procedure>

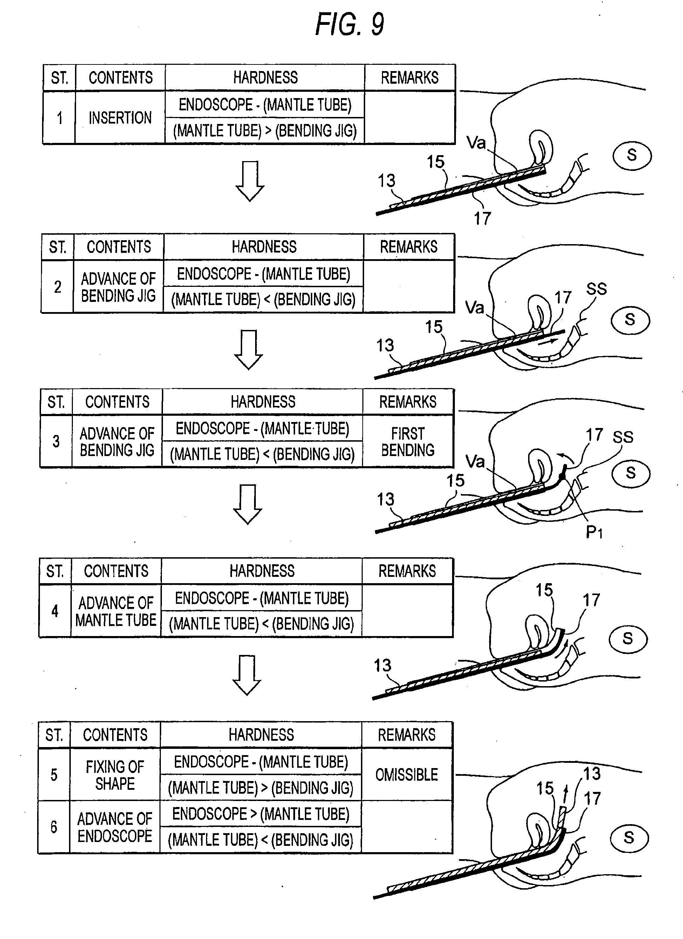

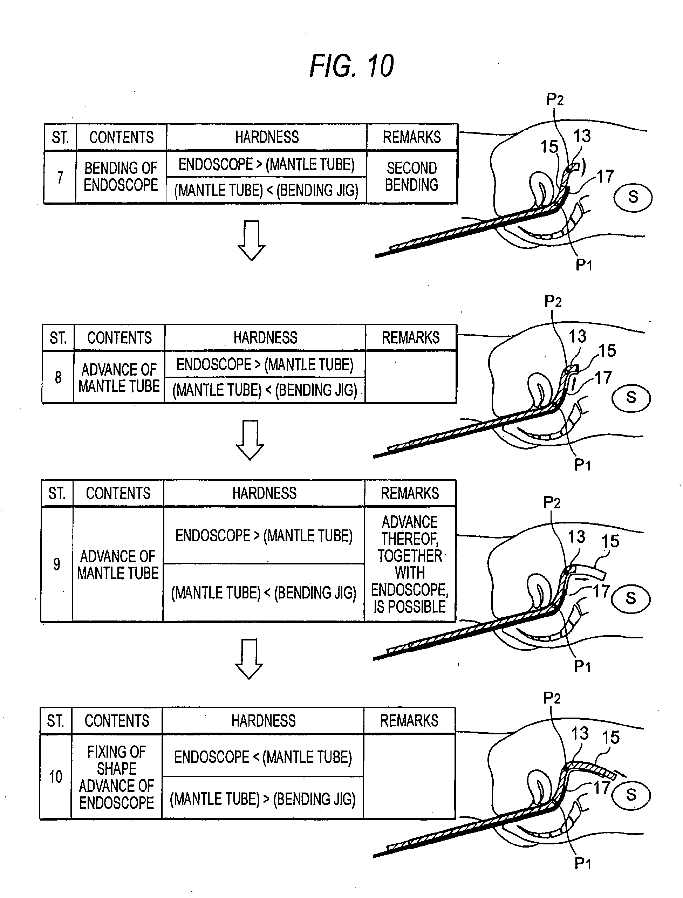

FIGS. 9 and 10 are explanatory diagrams illustrating a first insertion path securing procedure. In each of FIGS. 9 and 10, the relative bending rigidity (hereinafter referred to as hardness) relationship among the endoscope (endoscope insertion portion 13), the mantle tube 15, the bending jig 19 (bending sheath portion 17) is shown corresponding to each insertion step.

The hardness of the mantle tube 15 can be changed between the bendably flexible state and the rigid state in which the shape is fixed. The hardness of the bending jig is changed between a case in which the bending portion 65 illustrated in FIG. 6 by traction of the traction wire 69 is bent, and a normal state in which the traction is terminated. The hardness of the endoscope can be changed between a case in which the bending portion 53 illustrated in FIG. 5 is brought into a free state, and a case in which a bending angle of the bending portion 53 is specified by operating the angle knob 57 (including a state in which the bending angle is locked).

First, the procedure is described from step 1 illustrated in FIG. 9.

### (Step 1 Insertion)

In a state in which the endoscope insertion portion 13 and the bending sheath portion 17 are inserted into the mantle tube 15, the mantle tube 15 is brought into a rigid state in which the shape thereof is a linear shape, and the shape of the mantle tube 15 is fixed. The mantle tube 15 put into a linear shape is inserted into a patient's vagina Va. A leading end of the mantle tube 15 is advanced into the body-cavity from the posterior vaginal fornix (see FIG. 7A) punctured by the trocar. The hardness of the mantle tube 15 at that time is set to be larger than that of the bending sheath portion 17. Accordingly, a stable insertion operation is secured. The hardness of the mantle tube 15 is not particularly specified corresponding to the bending rigidity of the endoscope insertion portion 13. The endoscope insertion portion 13 can be inserted into the mantle tube 15 after the mantle tube 15 is inserted into the vagina Va. When the endoscope insertion portion 13 is inserted into the vagina Va while the endoscope insertion portion 13 is inserted into the mantle tube 15, the hardness of the mantle tube 15 can be lowered to a level close to that of the bending sheath portion 17.

### (Step 2 Advance of Bending Jig)

The hardness of the mantle tube 15 is set to be lower than that of the bending sheath portion 17. The bending sheath portion 17 is advanced into the body-cavity from the leading end of the mantle tube 15. The length of advance of the bending sheath portion 17 is confined to a level at which the bending sheath portion 17 does not reach the sacrum SS.

### (Step 3 Bending of Bending Jig)

The bending sheath portion 17 protruded from the mantle tube 15 is bent so that the sacrum SS located on an extension of the linearly-shaped mantle tube 15 is bypassed from the insertion path to be secured. This bending position is a first bending point P1 of the insertion path to be secured.

### (Step 4 Advance of Mantle Tube)

The mantle tube 15 is advanced to the leading end of the bending sheath portion 17 whose leading end is bent along the bending sheath portion 17. Thus, the mantle tube 15 is bent by the bending sheath portion 17. At that time, because the mantle tube 15 is bent in an area into which the endoscope insertion portion 13 is not inserted, a bending operation is easily performed.

### (Step 5 Fixing of Shape)

The hardness of the mantle tube 15 is set to be larger than that of the bending sheath portion 17 in a state in which the mantle tube 15 is bent at the first bending point P1.

### (Step 6 Advance of Endoscope)

The endoscope insertion portion 13 is advanced along the bent mantle tube 15. Thus, the endoscope insertion portion 13 is protruded from a leading end of the mantle tube 15. Because the endoscope insertion portion 13 is more flexible than the bending sheath portion 17, the shape fixing step, i.e., step 5 for increasing the hardness of the mantle tube 15 is omissible.

### (Step 7 Bending of Endoscope)

As illustrated in FIG. 10, the endoscope insertion portion 13 is bent such that the insertion path to be secured is directed to the stomach S. This bending position is a second bending point P2 of the insertion path to be secured. (At the second bending point P2, the endoscope insertion portion 13 can be bent in a state in which the endoscope insertion portion 13 together with the mantle tube 15 is advanced.) At that time, the bending sheath portion 17 continues to maintain the curved shape at the first bending point P1.

### (Step 8 Advance of Mantle Tube)

The leading end of the mantle tube 15 is advanced along the endoscope insertion portion 13 whose leading end is bent. Thus, the mantle tube 15 is bent by the endoscope insertion portion 13. The mantle tube 15 is deformed like a letter "S", in which first bending and second bending are performed. When a curved shape is formed in the mantle tube 15 at the second bending point P2, the bending sheath portion 17 maintains the curved shape due to the first bending at the first bending point P1. Thus, the mantle tube 15 does not lose the curved shape at the first bending point P1.

### (Step 9 Advance of Mantle Tube)

The mantle tube 15 is more advanced to a neighborhood position of the stomach S in a state in which the curved shape at the first bending point P1 is maintained by the bending sheath portion 17, and in which the curved shape at the second bending point P2 is maintained by the endoscope insertion portion 13.

### (Step 10 Fixing of Shape and Advance of Endoscope)

The shape of the mantle tube 15 is fixed by increasing the hardness of the mantle tube 15 bent like a letter "S" by the bending sheath portion 17 and the endoscope insertion portion 13. Consequently, the insertion path from the vagina Va to the stomach S is secured by the mantle tube 15. Then, the endoscope insertion portion 13 is more advanced. Thus, the leading end portion of the endoscope is made to approach the stomach S.

The above flexible state of the mantle tube 15 can be expressed as a state in which the mantle tube 15 can be bent by bending the bending sheath portion 17 in a condition in which the bending sheath portion 17 is disposed at a more distal side than the distal end of the mantle tube 15, and causing the mantle tube 15 to advance along the bent bending sheath portion 17. The above rigid state can be expressed as a state in which the mantle tube 15 can maintain the curved shape given thereto by the bending sheath portion 17 when the endoscope insertion portion 13 is inserted along the mantle tube 15.

According to the above first insertion path securing procedure, even when the insertion path has the first bending point P1 and the second bending point P2, the mantle tube 15 can always maintain the curved shapes established at the bending points P1 and P2 within the range of the mantle tube 15 until the mantle tube 15 is formed into a final curved shape. Accordingly, the curved shape of the mantle tube 15 at each of the bending points P1 and P2 can be prevented from being lost in the middle of formation of the curved shapes. That is, when the bending at the bending point P2 is performed, the control of the bending at the bending point P1 is not canceled. Consequently, the curved shape at each of the bending points P1 and P2 can accurately be maintained.

The present embodiment has a plurality of bending function members, such as the bending jig 19 (bending portion 65) and the endoscope 11 (bending portion 53). Thus, the bending function members can individually be controlled at different timings. For example, the curved shape of the bending function member, which is once set, can be reformed in the middle of forming the curved shape of the mantle tube 15. Consequently, even when a time-dependent change of the curved shape of the mantle tube 15 due to the bending sheath portion 17 occurs, a desired curved-shape can surely be maintained by optionally correcting the change of the curved shape thereof. In addition, e.g., a curvature-factor once set in the middle of formation of the curved shape of the mantle tube 15 can be more reduced or increased. Such a fine adjustment of the curved shape of the mantle tube 15 can easily been performed at each bending position in the middle of formation of the curved shape thereof.

Consequently, the curved shape of the mantle tube 15, which is controlled by the bending jig and the endoscope, can accurately be maintained even after the insertion path is secured. Accordingly, unnecessary contact with body-parts and organs surrounding the insertion path can be prevented.

After the insertion path is secured, the bending sheath portion 17 is withdrawn from the mantle tube 15. Consequently, the communication hole 23 (see FIG. 2) of the mantle tube 15, in which the bending sheath portion 17 is inserted, can be utilized for other uses, e.g., insertion of a catheter. The range of manipulations can be expanded utilizing a multi-lumen structure.

In the case of the configuration of the above mantle tube 15, the insertion path to be secured has two bending points, i.e., the first bending point P1 and the second bending point P2. Thus, only two bending function members, i.e., the bending jig 19 and the bending portion 53 of the endoscope (see FIG. 5) are used. The number of bending function members can be increased according to the number of necessary bending points. For example, the number of the communication holes 23 (see FIG. 2) in the mantle tube 15 can be increased from 2 to an optional number. In addition, the bending jig 19 is inserted into each of the communication holes23. Thus, the configuration of the mantle tube 15 can appropriately be changed.

When the bending portion 53 of the endoscope is used as a bending function member, the curved shape thereof can be adjusted while observed with the endoscope. The contents of the manipulation can be simplified, and an accurately curved shape can be formed. When a curved shape is formed by a plurality of bending jigs without using the bending portion 53 of the endoscope, the curved shape can more finely be adjusted. After the insertion path is secured, the bending jig 19 can be withdrawn from the mantle tube 15. Thus, the above effects due to the multi-lumen structure can also be obtained.

The above example is performed in the case of the transvaginal extraction. However, even in the case of a gastric or transrectal extraction, the above procedure can similarly be performed. In addition, even when the insertion path securing apparatus is inserted by, e.g., incising a lumen or the like, similar advantages can be obtained.

### <Second Insertion Path Securing Procedure>

Next, a second insertion path securing procedure is described.

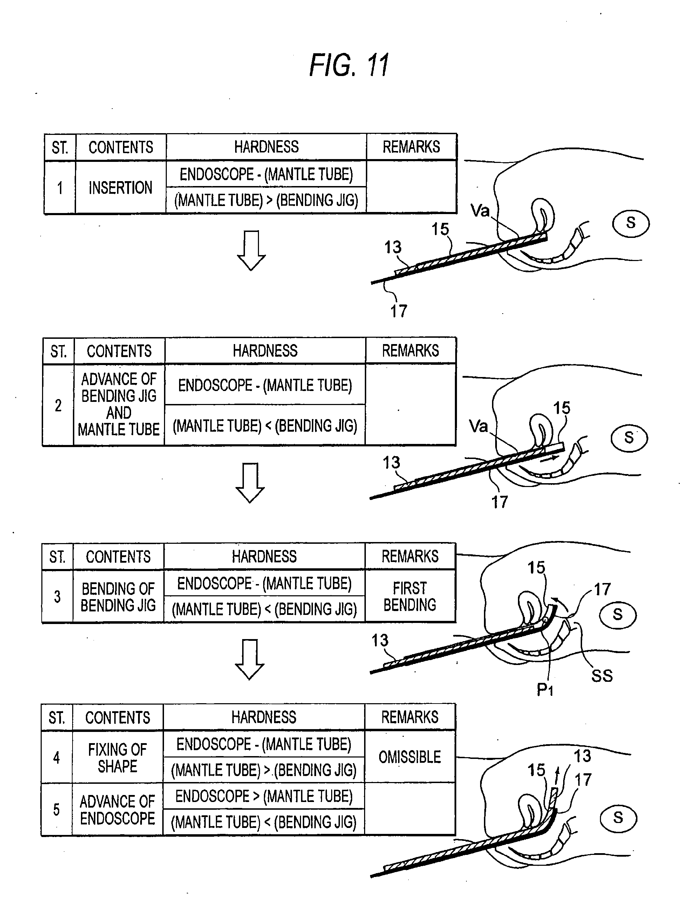

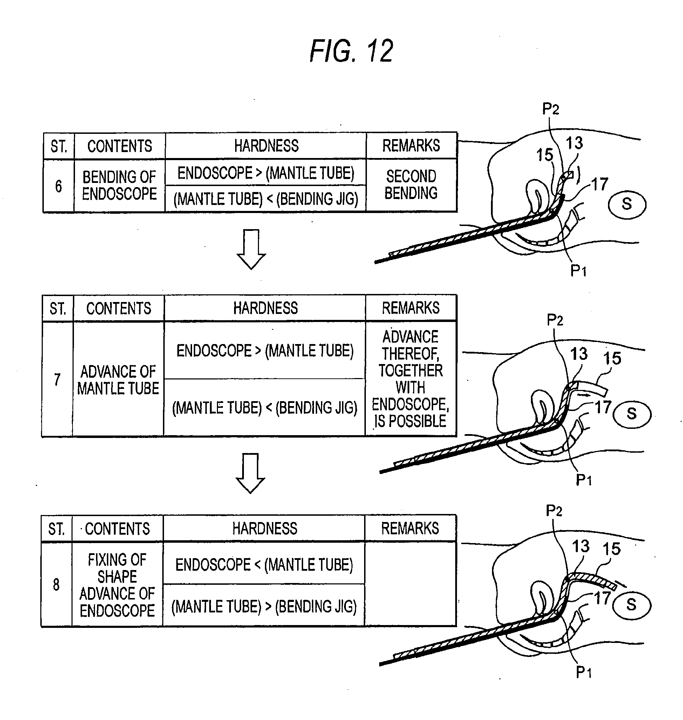

FIGS. 11 and 12 are explanatory diagrams illustrating the second insertion path securing procedure. First, the second insertion path securing procedure is described serially from step 1 illustrated in FIG. 11.

### (Step 1 Insertion)

Similarly to the above procedure, the mantle tube 15 into which the endoscope insertion portion 13 and the bending sheath portion 17 are inserted is formed into a linear shape and inserted into a patient's vagina Va. The leading end of the mantle tube 15 is advanced into the body-cavity from the posterior vaginal fornix. The hardness of the mantle tube 15 at that time is set to be larger than that of the bending sheath portion 17. Accordingly, a stable insertion operation is secured. The hardness of the mantle tube 15 is not particularly specified corresponding to the bending rigidity of the endoscope insertion portion 13. The endoscope insertion portion 13 can be inserted into the mantle tube 15 after the mantle tube 15 is inserted into the vagina Va. When the endoscope insertion portion 13 is inserted into the vagina Va while the endoscope insertion portion 13 is inserted into the mantle tube 15, the hardness of the mantle tube 15 can be lowered to a level close to that of the bending sheath portion 17.

### (Step 2 Advance of Bending Jig and Mantle Tube)

The hardness of the mantle tube 15 is set to be lower than that of the bending sheath portion 17. Then, the mantle tube 15 and the bending sheath portion 17 are advanced into the body-cavity. At that time, the endoscope insertion portion 13 is held at the position set in step 1.

### (Step 3 Bending of Bending Jig)

The bending sheath portion 17 protruded from a leading end of the endoscope insertion portion 13 is bent, together with the mantle tube 15, so as to bypass the sacrum SS. This bending position is a first bending point P1 of the insertion path to be secured.

### (Step 4 Fixing of Shape)

The hardness of the mantle tube 15 is set to be larger than that of the bending sheath portion 17, in a state in which the mantle tube 15 is bent at the first bending point P1.

### (Step 5 Advance of Endoscope)

The endoscope insertion portion 13 is advanced along the bent mantle tube 15. Thus, the endoscope insertion portion 13 is protruded from a leading end of the mantle tube 15. Because the endoscope insertion portion 13 is more flexible than the bending sheath portion 17, the shape fixing step, i.e., step 5 of increasing the hardness of the mantle tube 15 can be omitted.

### (Step 6 Bending of Endoscope)

As illustrated in FIG. 12, the endoscope insertion portion 13 is bent such that the insertion path to be secured is directed to the stomach S. This bending position is a second bending point P2 of the insertion path to be secured. At that time, the bending sheath portion 17 continues to maintain the curved shape at the first bending point P1.

### (Step 7 Advance of Mantle Tube)

The mantle tube 15 is advanced along the endoscope insertion portion 13 whose leading end is bent. Thus, the mantle tube 15 is bent by the endoscope insertion portion 13. The mantle tube 15 is deformed like a letter "S", in which first bending and second bending are performed. When a curved shape is formed in the mantle tube 15 at the second bending point P2, the bending sheath portion 17 maintains the curved shape due to the first bending at the first bending point P1. Thus, the mantle tube 15 does not lose the curved shape at the first bending point P1. Then, the mantle tube 15 is further advanced to the neighborhood position of the stomach S. (At the second bending point P2, the endoscope insertion portion 13 and the mantle tube 15 can be bent in a state in which both the endoscope insertion portion 13 and the mantle tube 15 are advanced thereto.)

### (Step 8 Fixing of Shape and Advance of Endoscope)

In a state in which the curved shape at the first bending point P1 is maintained by the bending sheath portion 17, and in which the curved shape at the second bending point P2 is maintained by the endoscope insertion portion 13, the shape of the mantle tube 15 is fixed by increasing the hardness of the mantle tube 15. Consequently, the insertion path from the vagina Va to the stomach S is secured by the mantle tube 15. Then, the endoscope insertion portion 13 is more advanced. Thus, the leading end portion of the endoscope is made to approach the stomach S.

The flexible state of the mantle tube 15 can be expressed as a state in which the mantle tube 15 can be bent by operating the bending sheath portion 17 in a condition in which the bending sheath portion 17 is disposed at a more proximal side than the distal end of the mantle tube 15. The rigid state can be expressed as a state in which the mantle tube 15 can maintain the curved shape given thereto by the bending sheath portion 17 when the endoscope insertion portion 13 is inserted along the mantle tube 15.

According to the above second insertion path securing procedure, when the curved shape of the mantle tube 15 is formed at the first bending point P1, the bending sheath portion 17 and the mantle tube 15 are integrally bent. Accordingly, the mantle tube 15, i.e., the insertion path can directly be bent, so that the curved shape can more accurately be set.

### <Third Insertion Path Securing Procedure>

Next, a third insertion path securing procedure is described.

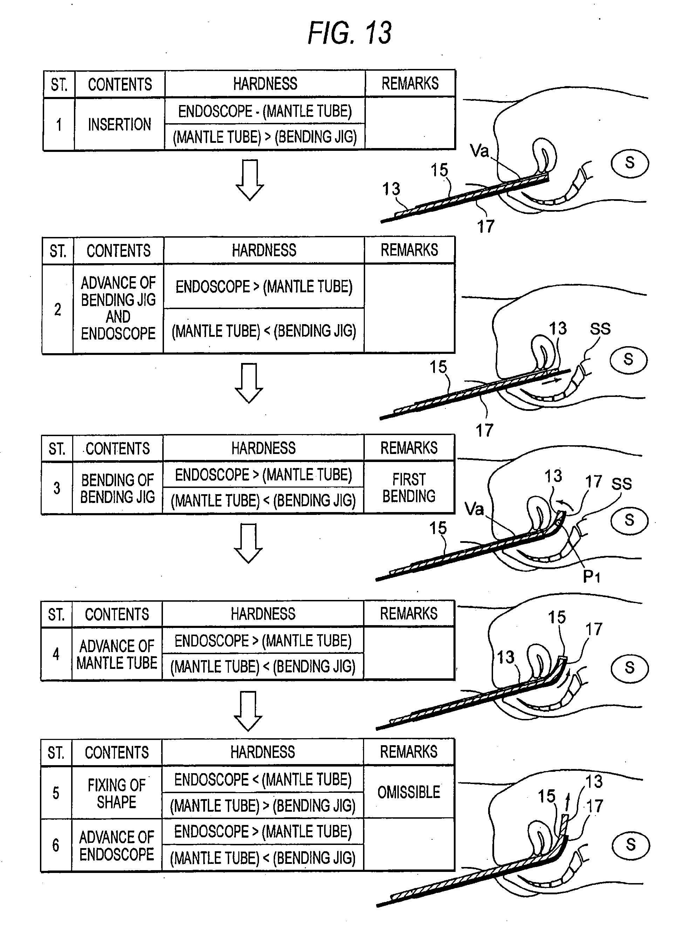

FIG. 13 is an explanatory diagram illustrating the third insertion path securing procedure.

### (Step 1 Insertion)

Similarly to the above procedure, the mantle tube 15 into which the endoscope insertion portion 13 and the bending sheath portion 17 are inserted is formed into a linear shape and inserted into a patient's vagina Va. The leading end of the mantle tube 15 is advanced into the body-cavity from the posterior vaginal fornix. The hardness of the mantle tube 15 at that time is set to be larger than at least that of the bending sheath portion 17. Accordingly, a stable insertion operation is secured. The hardness of the mantle tube 15 is not particularly specified corresponding to the bending rigidity of the endoscope insertion portion 13. The endoscope insertion portion 13 can be inserted into the mantle tube 15 after the mantle tube 15 is inserted into the vagina Va.

### (Step 2 Advance of Bending Jig and Advance of Endoscope)

The hardness of the mantle tube 15 is set to be lower than those of the endoscope insertion portion 13 and the bending sheath portion 17. Then, the endoscope insertion portion 13 and the bending sheath portion 17 are advanced into the body-cavity. At that time, the mantle tube 15 is held at the position set in step 1.

### (Step 3 Bending of Bending Jig)

The bending sheath portion 17 protruded from a leading end of the mantle tube 15 is bent to bypass the sacrum SS. This bending position is the first bending point P1 of the insertion path to be secured. A bending operation of the bending sheath portion 17 at that time can be performed under observation by the endoscope. Then, the endoscope insertion portion 13 is brought into substantially the same bent state as the bent state of the bending sheath portion 17 after the bending operation of the bending sheath portion 17.

### (Step 4 Advance of Mantle Tube)

The flexibilized mantle tube 15 to the leading end of the bending sheath portion 17, whose leading end is bent, and to that of endoscope insertion portion 13, whose leading end is bent, are advanced along the bending sheath portion 17 and the endoscope insertion portion 13. Thus, the mantle tube 15 is bent.

### (Step 5 Fixing of Shape)

The hardness of the mantle tube 15 is set to be larger than those of the endoscope insertion portion 13 and the bending sheath portion 17, in a state in which the mantle tube 15 is bent at the first bending point P1.

### (Step 6 Advance of Endoscope)

The endoscope insertion portion 13 is advanced. Thus, the endoscope insertion portion 13 is protruded from the leading end of the mantle tube 15. Because the endoscope insertion portion 13 is more flexible than the bending sheath portion 17, the shape fixing step, i.e., step 5 of increasing the hardness of the mantle tube 15 is omissible.

The subsequent steps 7 to 10 are similar to the associated steps of the first insertion path securing procedure, as illustrated in FIG. 10.

According to the above third insertion path securing procedure, when the insertion path is bent at the first bending point P1, the bent state thereof caused by the bending jig can be adjusted under observation by the endoscope. Accordingly, the curved shape at the first bending point P1 can more accurately be set, based on visual information. In addition, the manipulation can be simplified.

### <Fourth Insertion Path Securing Procedure>

Next, a fourth insertion path securing procedure is described.

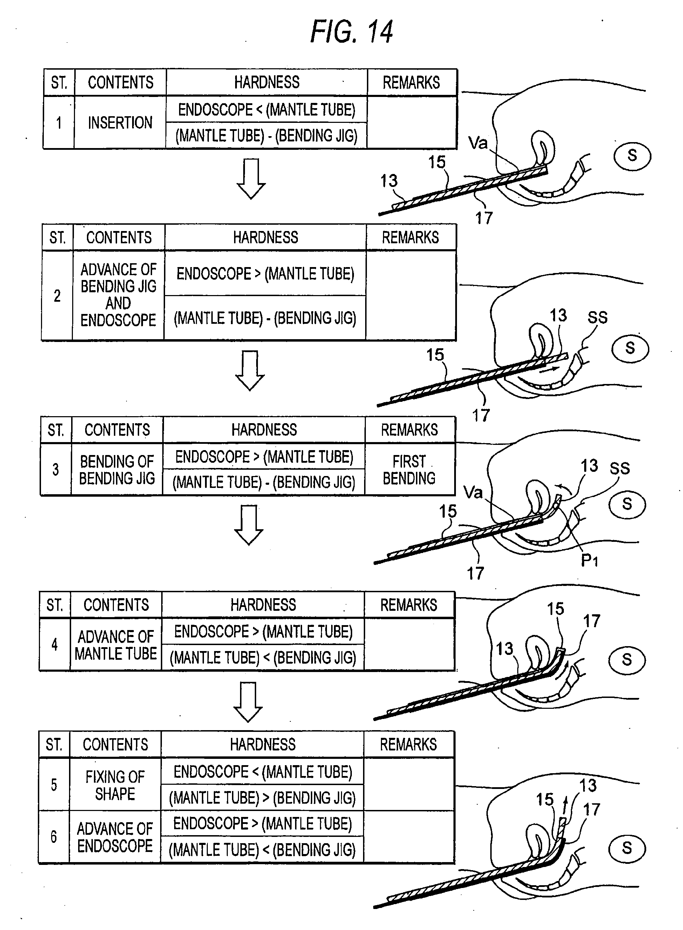

FIG. 14 is an explanatory diagram illustrating the fourth insertion path securing procedure.

### (Step 1 Insertion)

Similarly to the above procedure, the mantle tube 15 into which the endoscope insertion portion 13 and the bending sheath portion 17 are inserted is formed into a linear shape, brought into a rigid state, and inserted into a patient's vagina Va. The leading end of the mantle tube 15 is advanced into the body-cavity from the posterior vaginal fornix. The hardness of the mantle tube 15 at that time is set to be larger than at least that of the endoscope insertion portion 13. Accordingly, a stable insertion operation is secured. The hardness of the mantle tube 15 is not particularly specified corresponding to the bending rigidity of the endoscope insertion portion 13. The bending sheath portion 17 can be inserted into the mantle tube 15 after the mantle tube 15 is inserted into the vagina Va.

### (Step 2 Advance of Endoscope)

The hardness of the mantle tube 15 is set to be lower than that of the endoscope insertion portion 13. Then, the endoscope insertion portion 13 is advanced into the body-cavity. At that time, the mantle tube 15 and the bending sheath portion 17 are held at the position set in step 1.

### (Step 3 Bending of Endoscope)

A leading end of the endoscope insertion portion 13 protruded from a leading end of the mantle tube 15 is bent to bypass the sacrum SS. This bending position is the first bending point P1 of the insertion path to be secured. A bending operation of the endoscope insertion portion 13 at that time can be performed under observation by the endoscope. Thus, the adjustment of the bending is facilitated.

### (Step 4 Advance of Mantle Tube and Bending Jig)

The flexibilized mantle tube 15 and the flexibilized bending sheath portion 17 are advanced to the leading end of the endoscope insertion portion 13, whose leading end is bent, along the endoscope insertion portion 13. Thus, the mantle tube 15 is bent.

### (Step 5 Fixing of Shape)

The hardness of the mantle tube 15 is set to be larger than those of the endoscope insertion portion 13 and the bending sheath portion 17, in a state in which the mantle tube 15 is bent at the first bending point P1. At that time, the bending sheath portion 17 is caused to maintain the curved shape of the hardened mantle tube 15, i.e., the curved shape of the endoscope insertion portion 13 at the first bending point P1.

### (Step 6 Advance of Endoscope)

The hardness of the mantle tube 15 is set to be smaller than those of the endoscope insertion portion 13 and the bending sheath portion 17. Thus, the endoscope insertion portion 13 is advanced. In addition, the endoscope insertion portion 13 is protruded from the leading end of the mantle tube 15. Because the bending sheath portion 17 at that time maintains the curved shape formed at the first bending point P1, the curved shape formed at the first bending point P1 is not lost even when the mantle tube 15 is flexibilized. Because the endoscope insertion portion 13 is more flexible that the bending sheath portion 17, the shape fixing step, i.e., step 5 of increasing the hardness of the mantle tube 15 is omissible.

The subsequent steps 7 to 10 are similar to the associated steps of the first insertion path securing procedure, as illustrated in FIG. 10.

According to the above fourth insertion path securing procedure, the endoscope insertion portion 13 is advanced while the endoscope insertion portion 13 is always at the forefront of an insertion destination region. Thus, the insertion path can be determined by being always observed. Accordingly, the curved shape at the first bending point P1 and that at the second bending point P2 can more accurately be set based on visual information. In addition, the manipulation can be simplified.

### <Fifth Insertion Path Securing Procedure>

Next, a fifth insertion path securing procedure is described.

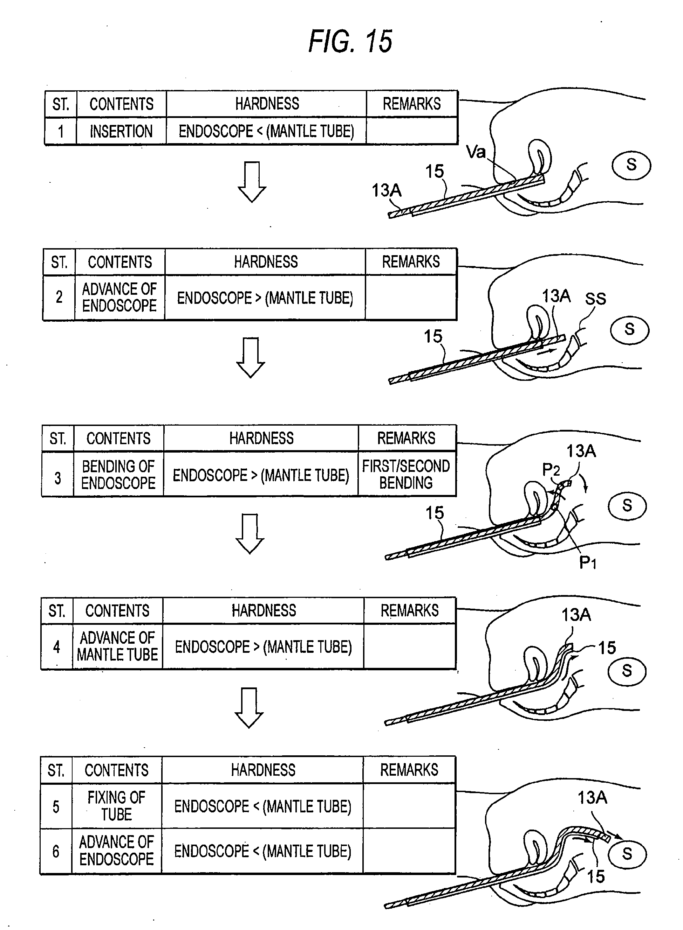

FIG. 15 is an explanatory diagram illustrating the fifth insertion path securing procedure. In this case, an endoscope is used, which can bend the endoscope insertion portion 13 illustrated in FIG. 5 like a letter "S". More specifically, the endoscope insertion portion 13 can be bent like a letter "S" by configuring the endoscope such that two bending portions 53 are provided at two places, and that the bending portions 53 are bent in directions respectively differing from each other by being operated from the bending operation portion 57.

### (Step 1 Insertion)

The mantle tube 15 into which an endoscope insertion portion 13A is inserted is brought into a rigid state in which the shape thereof is a linear shape. In addition, the mantle tube 15 is inserted into a patient's vagina Va. A leading end of the mantle tube 15 is advanced into the body-cavity from the posterior vaginal fornix. The hardness of the mantle tube 15 at that time is set to be larger than that of the endoscope insertion portion 13A. Accordingly, a stable insertion operation is secured.

### (Step 2 Advance of Endoscope)

The hardness of the mantle tube 15 is set to be lower than that of the endoscope insertion portion 13A. Then, the endoscope insertion portion 13A is advanced into the body-cavity. At that time, the mantle tube 15 is held at the position set in step 1.

### (Step 3 Bending of Endoscope)

A leading end of the endoscope insertion portion 13A protruded from a leading end of the mantle tube 15 is bent like a letter "S" to bypass the sacrum SS. Bending positions in this case are a first bending point P1 and a second bending point P2 of the insertion path to be secured. Because the bending operation is performed under observation by the endoscope, the adjustment of the bending thereof is facilitated.

### (Step 4 Advance of Mantle Tube)

The flexibilized mantle tube 15 to the leading end of the endoscope insertion portion 13A, whose leading end is bent like a letter "S", is advanced along the endoscope insertion portion 13A. Thus, the mantle tube 15 is bent like a letter "S".

### (Step 5 Fixing of Shape)

The hardness of the mantle tube 15 is set to be smaller than that of the endoscope insertion portion 13A, in a state in which the mantle tube 15 is bent at the first bending point P1 and the second bending point P2. At that time, the endoscope insertion portion 13A maintains the curved shapes thereof formed at the first bending point P1 and the second bending point P2. Thus, the curved shapes formed at the first bending point P1 and the second bending point P2, respectively, are not lost.

### (Step 6 Advance of Endoscope)

The endoscope insertion portion 13A into the body cavity is advanced by setting the bending portion 53 (see FIG. 5) into a free state. Thus, the leading end portion of the endoscope is caused to approach the stomach S.

According to the fifth insertion path securing procedure, the shape of the endoscope insertion portion 13A at each of the first bending point P1 and the second bending point P2 is matched at a time with the curved shape to be formed at an associated one of the first bending point P1 and the second bending point P2. Thus, the mantle tube 15 can be bent into a desired shape of the insertion path by a simple manipulation.

According to the insertion path securing procedure performed by each of the above insertion path securing apparatuses, when the mantle tube 15 is bent, the curved state of the mantle tube 15 at the position of each bending point is maintained by a plurality of bending members. Thus, the curved shape formed at each bending point can surely be maintained until the curved parts of the mantle tube 15 are completely formed. Accordingly, the shape of the insertion path can be prevented from changing from the set curved-shape.

Next, other configurations of the insertion path securing apparatus 100 are described below as modifications.

### <Modification 1>

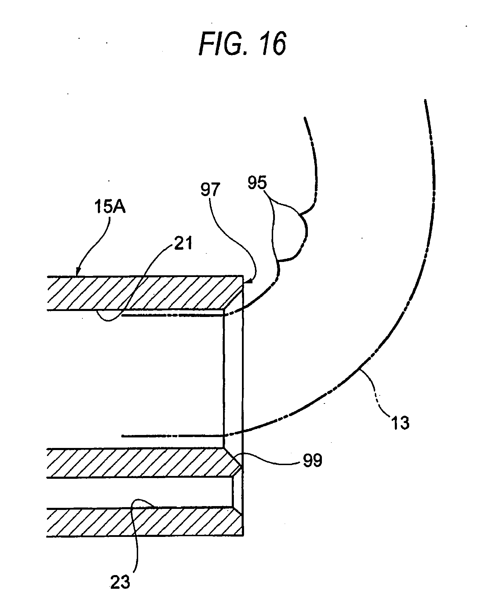

FIG. 16 is a cross-sectional diagram taken at a leading end portion at a side in which a mantle tube is inserted into a body-cavity. An endoscope insertion portion 13 is inserted into a guide hole 21 of a mantle tube 15A. The endoscope insertion portion 13 protruded from a body-cavity insertion side of the mantle tube 15A is bent in a desired direction. The surface of the endoscope insertion portion 13 is covered with a resin tube on which a crimp (ridge) 95 continuously extending in a circumferential direction is generated by bending the endoscope insertion portion 13. The crimp 95 abuts against the body-cavity insertion side end portion 97 of the guide hole 21 when the endoscope insertion portion 13 is inserted and removed into and from the guide hole 21. Thus, friction due to the engagement therebetween is liable to occur.

Thus, in the mantle tube 15A, a tapered portion 99A formed by increasing the inside diameter of the guide hole 21 towards the body-cavity insertion side end portion 97 is provided on the inner wall of the body-cavity insertion side end portion 97 of the guide hole 21. Consequently, when the bent endoscope insertion portion 13 is inserted and removed into and from the guide hole 21, the crimp 95 easily moves beyond the body-cavity insertion side end portion 97 due to the tapered portion 99A to eliminate the engagement therebetween. Accordingly, the insertion/removal of the endoscope insertion portion 13 can smoothly be performed. A user's maneuvering feeling of the endoscope can be improved.

### <Modification 2>

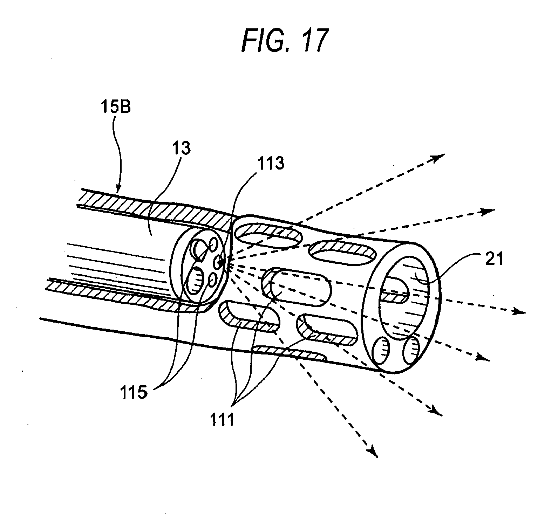

FIG. 17 is a partially cross-sectional perspective diagram taken at the leading end portion of a mantle tube, which is at the side in which the mantle tube is inserted into the body-cavity. The mantle tube 15B is such that a plurality of opening holes 111 communicating with the guide hole 21 are bored in the body-cavity insertion side outer circumferential surface thereof. Opening holes 111 serve as peep windows corresponding to an observation window 113 and illumination windows 115 formed in the leading end portion of the endoscope insertion portion 13. Thus, surrounding areas can be observed from a near-side position from which a leading end of the endoscope insertion portion 13 is protruded from the guide hole 21.

With this configuration, an endoscope observation from the guide hole 21 can be performed, even if the mantle tube 15B is not formed of a translucent material. Thus, the degree of flexibility of selecting the material of the mantle tube 15B is enhanced. If the mantle tube 15B is formed of a translucent material, when an endoscope observation from the guide hole 21 is performed, high-visibility images of surrounding areas can be obtained through the opening holes 111.

### <Modification 3>

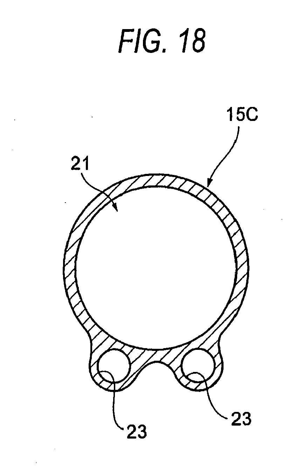

FIG. 18 is a cross-sectional diagram illustrating a mantle tube. The mantle tube according to the invention is not limited to that formed cross-sectionally, as illustrated in FIG. 3. A mantle tube according to the invention can be formed thin cross-sectionally, as illustrated in FIG. 18. That is, a mantle tube 15C is configured such that communication holes 23, 23 are individually arranged on an outer circumferential side of a guide hole 21, and that the outer circumferential part of the guide hole 21 is reduced in thickness. With this configuration, the diameter of the mantle tube can be reduced. Thus, the mantle tube can be advanced into a narrower hole in a body-cavity. Although the communication holes 23, 23 are respectively provided at two places in the mantle tube 15C in an example illustrated in FIG. 18, another communication hole can be provided therein.

### <Modification 4>

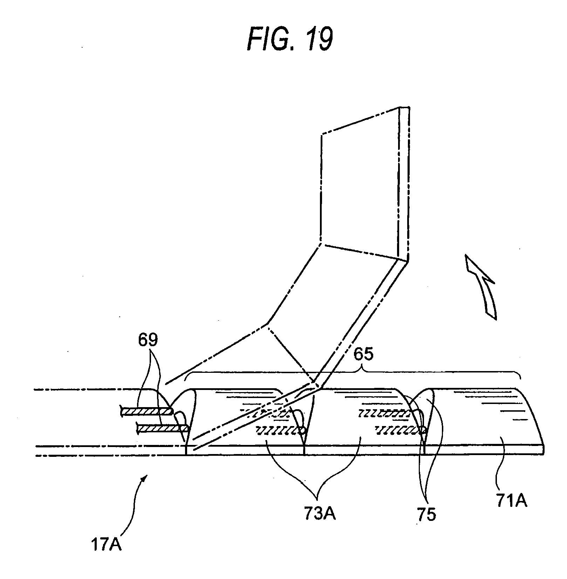

FIG. 19 is a perspective diagram illustrating a leading end portion of a bending sheath member of a bending jig. This modification employs a bending sheath member 17A, instead of the above bending sheath portion 17 shaped cross-sectionally like a circle. The bending sheath member 17A is an elongated-and-curved-plate-like member configured such that the front and rear surfaces thereof are shaped cross-sectionally like a circular-arc, and that a traction wire 69 is inserted into the plate-like member with a certain thickness, which extends in a longitudinal direction.

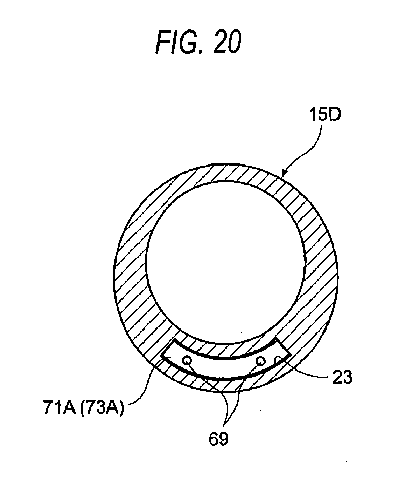

The bending portion 65 provided at a leading end of the bending sheath portion 17A includes a leading end piece 71A and a plurality of bending pieces 73A provided in a connected-row-arrangement. An end surface that each of the pieces faces is provided with a cutout portion 75 which is obliquely cutaway and similar to the cutout portion of the above embodiment. When a traction wire 69 is pulled, the bending portion 65 performs a bending operation due to the cutout portion 75. The bending sheath portion 17A is inserted into a communication hole 23 of a mantle tube 15D, which is shaped cross-sectionally as illustrated in FIG. 20. The mantle tube 15D is bent by the bending sheath portion 17A.

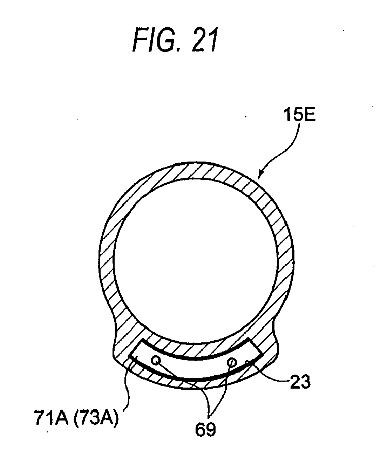

The number of communication holes 23 to be formed in the mantle tube 15D is not limited to 1 in the case illustrated in FIG. 20. This modification is configured such that communication holes 23 are provided at a plurality of places. Alternatively, as illustrated in FIG. 21, this modification can be configured such that the outer circumferential part of the guide hole 21 is reduced in thickness to reduce the thickness of the mantle tube 15 and to facilitate the bending of the mantle tube 15.

### <Modification 5>

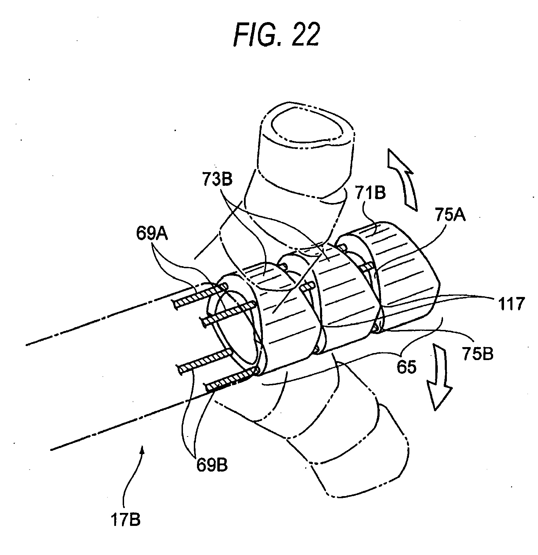

FIG. 22 is a perspective diagram illustrating a leading end portion of a bending sheath member of a bending jig. A bending sheath portion 17B is employed, instead of the above bending sheath portion 17 shaped cross-sectionally like a circle. The bending sheath portion 17B is an elongated cylindrical member that is shaped cross-sectionally like a circle and that has a leading end portion bendable in both of an upward direction and a downward direction, as viewed in FIG. 22. A bending portion 65 provided at a leading end of the bending sheath portion 17B includes a leading end piece 71B and a plurality of bending pieces 73B provided in a connected-row-arrangement. Adjacent pieces of each pair abut against each other at the circumferential positions of two points (an abutment point 117 at a near side, as viewed in FIG. 22, and another abutment point (not shown) located in a depth direction) arranged in a direction of a diameter. A pair of cutout portions 75A and 75B makes the bending portion 65 bendable in a plane perpendicular to the direction of a diameter thereof. The cutout portions 75A and 75B of each pair are formed on end portions of each of the pieces.

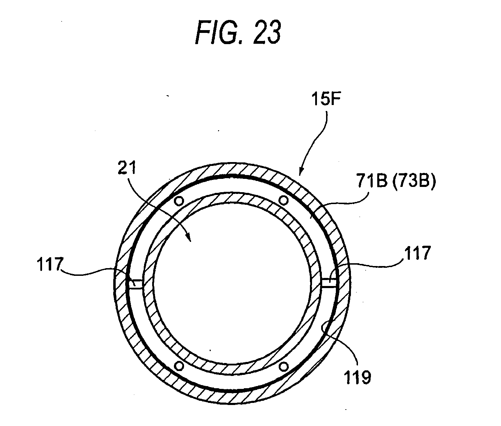

That is, when the bending portion 65 is bent in an upward direction, as viewed in FIG. 22, an upper traction wire 69A is pulled. When the bending portion 65 is bent in a downward direction, a lower traction wire 69B is pulled. The bending sheath portion 17B is inserted into a communication hole 119 of a mantle tube 15F cross-sectionally illustrated in FIG. 23. Thus, the mantle tube 15F is bent.

The configuration of the mantle tube is not limited to the above configuration in which the hardness thereof is variable between the flexible state of the flexibilized mantle tube and the rigid state in which the shape thereof is fixed. The mantle tube can be configured such that a third lumen is provided in addition to the guide hole (first lumen) and the communication hole (second lumen), that an elongated hardness variable member whose hardness is variable according to the principle similar to that in the case of the mantle tube of the above embodiment is inserted into the third lumen to change the hardness of the mantle tube according to the present modification. In this case, it is unnecessary to impart, to the mantle tube 15 itself, the functions of making the hardness thereof variable, so that the configuration of the mantle tube can be simplified. The configuration of the mantle tube according to the invention is not limited to the configuration in which a bending member is provided in the inside of the mantle tube. The mantle tube according to the invention can be configured such that a bending member is disposed on the exterior of the mantle tube.

The invention is not limited to the above embodiments. The invention is intended to be susceptible to modifications and applications made by those skilled in the art based on the descriptions of the specification and known technology. The modifications and the applications are included within a scope of protection.

As described above, the present specification discloses the following matters.

[1] It is an insertion path securing apparatus for guiding an insertion instrument to be inserted into a body-cavity by using an insertion portion having a longitudinal axis. The insertion path securing apparatus includes a first bending member, a second bending member and a hardness changing member. The first bending member is arranged advanceably and withdrawably along the longitudinal axis of the insertion portion. The first bending member has a first bending action portion provided at a distal side and a first bending operation portion provided at a proximal side, and can bend the first bending action portion by operating the first bending operation portion. The second bending member is arranged advanceably and withdrawably along the longitudinal axis of the insertion portion. The second bending member has a second bending action portion provided at a distal side and a second bending operation portion provided at a proximal side, and can bend the second bending action portion at a position on the longitudinal axis, which differs from a position corresponding to the first bending member, by operating the second bending operation portion. The hardness changing member is arranged along the longitudinal axis of the insertion portion, has a hardness variable portion provided at a distal side and a hardness changing operation portion provided at a proximal side. The hardness variable portion is configured to be able to change hardness between a flexible state and a rigid state by operating the hardness changing operation portion. In the flexible state, flexibility is imparted to the insertion portion by operating the hardness changing operation portion to be able to bend the insertion portion by the first bending member or the second bending member. In the rigid state, a curved shape imparted to the insertion portion by the first bending member or the second bending member is maintained.

According to this insertion path securing apparatus, when parts of the insertion portion, which correspond to different positions on a longitudinal axis, are bent by the first bending member and the second bending member, respectively, the insertion path can be formed by surely maintaining the curved shapes formed at the bending positions.

[2] The insertion path securing apparatus according to [1], the flexible state of the hardness changing member is a state in which the hardness changing member can be bent by causing the first bending action portion to bend the first bending action portion when the first bending action portion is arranged at a more distal side than a distal end of the hardness changing member, and by causing the hardness changing member to advance along the bent first bending action portion. The rigid state is a state in which the insertion portion can maintain a curved shape imparted thereto by the first bending member when the second bending member is inserted along the hardness changing member.

According to this insertion path securing apparatus, the hardness changing member in the flexible state is bent by being advanced along the first bending action portion. After bent, the hardness changing member is brought into a rigid state. Thus when the second bending member is inserted along the hardness changing member, the curved shape imparted by the first bending member to the hardness changing member can be maintained.

[3] The insertion path securing apparatus according [1], the flexible state of the hardness changing member is a state in which the hardness changing member can be bent by operating the first bending operation portion when the first bending action portion is arranged at a more proximal side than a distal end of the hardness changing member. The rigid state is a state in which the insertion portion can maintain a curved shape imparted thereto by the first bending member when the second bending member is inserted along the hardness changing member.

According to this insertion path securing apparatus, the hardness changing member in a flexible state is bent by performing the bending operation of the first bending action portion. After bent, the hardness changing member is brought into a rigid state. Thus, when the second bending member is inserted along the hardness changing member, the curved shape imparted by the first bending member to the hardness changing member can be maintained.

[4] The insertion path securing apparatus according to one of [1] to [3], the hardness changing member includes a state maintaining unit configured to maintain the flexible state or the rigid state.

According to this insertion path securing apparatus, the hardness changing member can be maintained in a flexible state or rigid state. The operation for securing the insertion path can be simplified.

[5] The insertion path securing apparatus according to one of [1] to [4], each of the first bending member and the second bending member includes a condition maintaining unit configured to maintain a curved shape.

According to this insertion path securing apparatus, the first bending member and the second bending member can maintain the curved shapes once set. The operation for securing the insertion path can be simplified.

[6] The insertion path securing apparatus according to one of [1] to [5], the first bending member and the second bending member are provided inside the hardness changing member.

According to this insertion path securing apparatus, the hardness changing member can be reduced in diameter without causing the first bending member and the second bending member to protrude to the outside of the hardness changing member.

[7] The insertion path securing apparatus according to one of [1] to [5], the first bending member and the second bending member are provided outside the hardness changing member.

According to this insertion path securing apparatus, the first bending member and the second bending member are disposed outside the hardness changing member.

[8] The insertion path securing apparatus according to one of [1] to [7], the hardness changing member is a mantle tube having a first lumen into which the first bending member is removably insertable, and a second lumen into which the second bending member is removably insertable. At least one of the first bending member and the second bending member is an endoscope having an observation unit.

According to this insertion path securing apparatus, the curved shape can be formed while observation is performed by the endoscope. Thus, the insertion path can accurately and surely be secured. In addition, the manipulation can be simplified.

[9] It is a mantle tube having an operating portion provided at a base-end side of an insertion portion having a longitudinal axis. The mantle tube includes a first lumen, a second lumen and a third lumen. A first bending member can be provided in the first lumen to have a first bending action portion provided at a distal side and a first bending operation portion provided at a proximal side, and to be able to bend the first bending action portion by operating the first bending operation portion. A second bending member can be provided in the second lumen to have a second bending action portion provided at a distal side and a second bending operation portion provided at a proximal side, and to be able to bend the second bending action portion at a position differing from that of the first bending member on the longitudinal axis by operating the second bending operation portion. A hardness changing member can be provided in the third lumen to have a hardness variable portion provided at a distal side and a hardness changing operation portion provided at a proximal side. The hardness variable portion is configured to be able to change hardness between a flexible state and a rigid state by operating the hardness changing operation portion. In the flexible state, flexibility is imparted to the insertion portion by operating the hardness changing operation portion to be able to bend the insertion portion by the first bending member or the second bending member. In the rigid state, a curved shape imparted to the insertion portion by the first bending member or the second bending member is maintained.

According to this mantle tube, the mantle tube can be bent at different positions on the longitudinal axis by the first bending member to be inserted into the first lumen, and the second bending member to be inserted into the second lumen. In addition, the fixing/unfixing of the shape of the mantle tube can be performed by the hardness changing member to be inserted in the third lumen. Thus, the shape of the mantle tube, which is formed by each of the first bending member and the second bending member, can be maintained. Consequently, the curved shape at each bending position on the mantle tube can accurately by formed, without losing the curved shape formed at each bending position.

[10] It is a mantle tube having an operating portion provided at a base-end side of an insertion portion having a longitudinal axis. The mantle tube includes a first lumen and a second lumen. A first bending member can be removably provided in the first lumen to have a first bending action portion provided at a distal side and a first bending operation portion provided at a proximal side, and to be able to bend the first bending action portion by operating the first bending operation portion. A second bending member can be removably provided in the second lumen to have a second bending action portion provided at a distal side and a second bending operation portion provided at a proximal side, and to be able to bend the second bending action portion at a position differing from that of the first bending member on the longitudinal axis by operating the second bending operation portion. The insertion portion is a mantle tube whose hardness can be changed between a flexible state and a rigid state by operating the hardness changing operation portion. In the flexible state, the insertion portion has flexibility so that the insertion portion can be bent by the first bending member or the second bending member. In the rigid state, a curved shape imparted to the insertion portion by the first bending member or the second bending member is maintained.

According to this mantle tube, the flexibilized mantle tube can be bent individually at different positions on the longitudinal axis by the first bending member to be inserted into the first lumen, and the second bending member to be inserted into the second lumen. In addition, the shape of the mantle tube, which is formed by each of the first bending member and the second bending member, can be maintained by changing the hardness of the mantle tube to that in the flexible state thereof. Accordingly, the curved shapes of parts of the mantle tube, which are respectively formed by the first bending member and the second bending member, can be maintained. Consequently, the curved shape at each bending position on the mantle tube can accurately by formed, without losing the curved shape at each bending position.
(A-1) The insertion path securing apparatus, wherein at least a body-cavity insertion side part of the mantle tube is made of a translucent material.

According to this insertion path securing apparatus, when the endoscope is inserted into the mantle tube, the inside of the body-cavity can be observed from a near side at which a leading end portion of the endoscope is protruded from the insertion-side end portion of the mantle tube.
(A-2) The insertion path securing apparatus, wherein a plurality of opening holes communicating with the guide hole are bored in at least an outer circumferential surface of a body-cavity insertion side part of the mantle tube.

According to this insertion path securing apparatus, when the endoscope is inserted into the mantle tube, the inside of the body-cavity can be observed through the opening hole.
(A-3) The insertion path securing apparatus, wherein a tapered portion, in which an inside diameter of the guide hole increases towards the body-cavity insertion side end portion, is formed in the inner wall of the body-cavity insertion side end portion of the guide hole.

According to this insertion path securing apparatus, when an insertion instrument inserted into the guide hole is inserted and removed into and from the guide hole, a protrusion (e.g., a crimp) formed on a surface of the insertion instrument easily moves therebeyond due to the tapered portion to eliminate the engagement therebetween. Accordingly, the insertion/removal of the insertion instrument can smoothly be performed. A user's maneuvering feeling can be improved.
(A-4) The insertion path securing apparatus including a valve element provided at a body-cavity insertion side end portion of at least one of the guide hole and the communication hole so that an elastic element extends from an inner wall surface to the center of the hole.

According to this insertion path securing apparatus, air is blocked by the valve element from flowing into and out of a duct in each hole. In addition, this insertion path securing apparatus can prevent change in the intra-abdominal pressure in the body-cavity that is an insertion destination.
(B-1) An insertion path securing method for guiding an insertion instrument to be inserted into a body-cavity, using an insertion path securing apparatus configured to have an elongated mantle tube into which an insertion instrument is inserted, and to be variable in bending-rigidity between a pliably flexible state and a rigid state in which a shape is fixed, and a plurality of bending function members having configured to be insertable along a longitudinal direction of the mantle tube and to include a bendable bending portion for bending the mantle tube. The insertion path securing method includes at least steps of causing a first one of the bending function members to protrude from the mantle tube brought into the flexible state and to bend, causing the mantle tube to advance by performing relative movement along the first one of the bending function members, causing a second one of the bending function members to advance by performing relative movement along the mantle tube while a curved shape of the first one of the bending function members is fixed, and causing the advanced second one of the bending function members to bend the mantle tube at a position in a longitudinal direction, which differs from a bending position at which bending by the first one of the bending function members is performed.
(B-2) An insertion path securing method for guiding an insertion instrument to be inserted into a body-cavity, using an insertion path securing apparatus configured to have an elongated mantle tube into which an insertion instrument is inserted, and to be variable in bending-rigidity between a bendably flexible state and a rigid state in which a shape is fixed, and a plurality of bending function members having configured to be insertable along a longitudinal direction of the mantle tube and to include a bendable bending portion for bending the mantle tube. The insertion path securing method includes at least steps of causing both of the mantle tube brought into the flexible state, and a first one of the bending function members to advance, causing the mantle tube to be bent by the first one of the bending function members, causing a second one of the bending function members to advance by performing relative movement along the mantle tube while a curved shape of the first one of the bending function members is fixed, and causing the advanced second one of the bending function members to bend the mantle tube at a position in a longitudinal direction, which differs from a bending position at which bending by the first one of the bending function members is performed.
(B-3) An insertion path securing method for guiding an insertion instrument to be inserted into a body-cavity, using an insertion path securing apparatus configured to have an elongated mantle tube into which an insertion instrument is inserted, and to be variable in bending-rigidity between a bendably flexible state and a rigid state in which a shape is fixed, and a plurality of bending function members having configured to be insertable along a longitudinal direction of the mantle tube and to include a bendable bending portion for bending the mantle tube. The insertion path securing method includes at least steps of causing a first one of the bending function members to protrude from the mantle tube brought into the flexible state and to bend, causing the mantle tube to advance by performing relative movement along the first one of the bending function members, causing a second one of the bending function members to advance by performing relative movement along the mantle tube, while a curved shape of the first one of the bending function members is fixed, to move the second one of the bending function members to a position of the first one of the bending function members, causing the first one of the bending function member to advance by performing relative movement with respect to the second one of the bending function member while causing the advanced second one of the bending function members to maintain a curved shape due to the first one of the bending function members, and causing the advanced first one of the bending function members to bend the mantle tube at a position in a longitudinal direction, which differs from a bending position at which bending by the second one of the bending function members is performed.
(B-4) The insertion path securing method according to one of (B-1) to (B-3) further including steps of arranging at least a third one of the bending function members to be able to advance and retreat in a longitudinal direction of the mantle tube, and causing the third one of the bending function members to bend the mantle tube at a position in the longitudinal direction thereof, which differs from a bending position at which bending is performed by the second one of the bending function member.
(B-5) The insertion path securing method according to one of (B-1) to (B-4) further including a step of changing, while the mantle tube is bent by the bending function members, the state of the mantle tube into a rigid state.
(B-6) A surgery method including steps of performing incision on a body-cavity wall tube or a body-surface, securing an insertion path of an insertion instrument, which extends from a small hole formed by the incision to a diseased part, using one of the insertion path securing methods according to (B-1) to (B-5), and performing treatment on the diseased part through the secured insertion path.

## Claims

1. An insertion path securing apparatus for guiding an insertion instrument to be inserted into a body-cavity by using an insertion portion having a longitudinal axis, the insertion path securing apparatus comprising:
a first bending member (i) that is arranged advanceably and withdrawably along the longitudinal axis of the insertion portion, (ii) that has a first bending action portion provided at a distal side and a first bending operation portion provided at a proximal side, and (iii) that can bend the first bending action portion by operating the first bending operation portion;
a second bending member (iv) that is arranged advanceably and withdrawably along the longitudinal axis of the insertion portion, (v) that has a second bending action portion provided at a distal side and a second bending operation portion provided at a proximal side, and (vi) that can bend the second bending action portion at a position on the longitudinal axis, which differs from a position corresponding to the first bending member, by operating the second bending operation portion; and
a hardness changing member (vii) that is arranged along the longitudinal axis of the insertion portion, (viii) that has a hardness variable portion provided at a distal side and a hardness changing operation portion provided at a proximal side,
wherein the hardness variable portion is configured to be able to change hardness between a flexible state and a rigid state by operating the hardness changing operation portion,
wherein in the flexible state, flexibility is imparted to the insertion portion by operating the hardness changing operation portion to be able to bend the insertion portion by the first bending member or the second bending member, and
wherein in the rigid state, a curved shape imparted to the insertion portion by the first bending member or the second bending member is maintained.

2. The insertion path securing apparatus according to claim 1,
wherein the flexible state of the hardness changing member is a state in which the hardness changing member can be bent by causing the first bending action portion to bend the first bending action portion when the first bending action portion is arranged at a more distal side than a distal end of the hardness changing member, and by causing the hardness changing member to advance along the bent first bending action portion, and
wherein the rigid state is a state in which the insertion portion can maintain a curved shape imparted thereto by the first bending member when the second bending member is inserted along the hardness changing member.

3. The insertion path securing apparatus according to claim 1,
wherein the flexible state of the hardness changing member is a state in which the hardness changing member can be bent by operating the first bending operation portion when the first bending action portion is arranged at a more proximal side than a distal end of the hardness changing member, and
wherein the rigid state is a state in which the insertion portion can maintain a curved shape imparted thereto by the first bending member when the second bending member is inserted along the hardness changing member.

4. The insertion path securing apparatus according to any one of claims 1 to 3,
wherein the hardness changing member includes a state maintaining unit configured to maintain the flexible state or the rigid state.

5. The insertion path securing apparatus according to any one of claims 1 to 4,
wherein each of the first bending member and the second bending member includes a condition maintaining unit configured to maintain a curved shape.

6. The insertion path securing apparatus according to any one of claims 1 to 5,
wherein the first bending member and the second bending member are provided inside the hardness changing member.

7. The insertion path securing apparatus according to any one of claims 1 to 5,
wherein the first bending member and the second bending member are provided outside the hardness changing member.

8. The insertion path securing apparatus according to any one of claims 1 to 7,
wherein the hardness changing member is a mantle tube having a first lumen into which the first bending member is removably insertable, and a second lumen into which the second bending member is removably insertable, and
wherein at least one of the first bending member and the second bending member is an endoscope having an observation unit.

9. A mantle tube comprising:
an operating portion that is provided at a base-end side of the insertion portion according to any one of claims 1 to 8,
a first lumen in which the first bending member according to any one of claims 1 to 8 can be provided;
a second lumen in which the second bending member according to any one of claims 1 to 8 can be provided; and
a third lumen in which the hardness changing member according to any one of claims 1 to 8 can be provided.

10. An insertion path securing method for guiding an insertion instrument to be inserted into a body-cavity, by using an insertion path securing apparatus configured to have an elongated mantle tube into which an insertion instrument is inserted, and to be variable in bending-rigidity between a pliably flexible state and a rigid state in which a shape is fixed, and a plurality of bending function members having configured to be insertable along a longitudinal direction of the mantle tube and to include a bendable bending portion for bending the mantle tube, the insertion path securing method comprising:
causing a first one of the bending function members to protrude from the mantle tube brought into the flexible state and to bend;
causing the mantle tube to advance by performing relative movement along the first one of the bending function members;
causing a second one of the bending function members to advance by performing relative movement along the mantle tube while a curved shape of the first one of the bending function members is fixed; and
causing the advanced second one of the bending function members to bend the mantle tube at a position in a longitudinal direction, which differs from a bending position at which bending by the first one of the bending function members is performed.

11. The insertion path securing method according to claim 10 further comprising:
arranging at least a third one of the bending function members to be able to advance and retreat in a longitudinal direction of the mantle tube; and
causing the third one of the bending function members to bend the mantle tube at a position in the longitudinal direction thereof, which differs from a bending position at which bending is performed by the second one of the bending function member.

12. The insertion path securing method according to claim 10 further comprising:
changing, while the mantle tube is bent by the bending function members, the state of the mantle tube into a rigid state.
